# EUROPÄISCHE PATENTSCHRIFT

(11) **EP 0 867 197 B1**
(45) Veröffentlichungstag und Bekanntmachung des Hinweises auf die Patenterteilung: **01.10.2003**
(21) Anmeldenummer: 98810214.1
(22) Anmeldetag: 13.03.1998
(51) Int. Cl.: A61M 39/02

(54) **Implantierbare Vorrichtung zur Arzneimittelverabreichung**
Implantable drug delivery device
Dispositif implantable pour l'administration de médicaments

(30) Priorität: 26.03.1997 CH 72997
(43) Veröffentlichungstag der Anmeldung: 30.09.1998
(73) Patentinhaber: Disetronic Licensing AG, 3401 Burgdorf (CH)
(72) Erfinder: Bestetti, G.E., Prof. Dr., 3098 Köniz (CH); Frei, Thomas, 3432 Lützelflüh (CH); Reinmann, Andreas, 3014 Bern (CH); Piller, Daniel, 3014 Bern (CH)
(74) Vertreter: Schwabe - Sandmair - Marx

(56) Entgegenhaltungen:
- GB-A- 2 000 977
- US-A- 3 783 868
- US-A- 4 321 914
- US-A- 5 098 398

## Beschreibung

Die Erfindung bezieht sich auf einen implantierbaren Portkörper zur Verabreichung von Arzneimittel gemäss dem Oberbegriff des Anspruchs 1.

Aus der Patentschrift US-A-5 306 255 ist ein subcutan implantierbarer Portkörper bekannt. Ein subcutaner Portkörper liegt vollkommen unter der Haut und verbleibt normalerweise mehrere Monate oder sogar Jahre im menschlichen Körper. Im Inneren des Portkörpers befindet sich die Portkammer. Die sich vollständig im menschlichen oder tierischen Körper befindende Portkammer wird hautseitig durch eine Durchstechmembran aus Kunststoff abgeschlossen, während gegen das Körperinnere ein Katheter befestigt ist, welcher zum Ausschüttungsort für Arzneimittel führt. Zur Arzneimittelverabreichung werden Haut und Membrane mit einer Nadel eines Infusionssets durchstochen. Dadurch entsteht ein durchgängiger Arzneimittelkanal, welcher vom Infusionsset bis zum Ausschüttungsort reicht.
Aus der Patentschrift EP-B-0 302 076 ist ein zylindrischer, percutan implantierbarer Portkörper bekannt. Im Gegensatz zum subcutanen Portkörper wird der percutane Portkörper nicht vollständig in einen menschlichen Körper implantiert, sondern derart im Gewebe befestigt, dass zumindest eine bestimmte Fläche des Portkörpers nicht von Haut überdeckt wird. Im Zentrum dieser Fläche befindet sich eine erste Öffnung. Eine zweite Öffnung des Portkörpers befindet sich gegenüber der ersten Öffnung, im vollständig von Gewebe umgebenen Bereich des Portkörpers. An dieser Öffnung ist ein Katheter angeschlossen, dessen Ende sich an demjenigen Ort innerhalb des Körpers befindet, an den das Arzneimittel transportiert werden soll. Der Portkörper besteht aus zwei ineinandergeschraubten Metallteilen. Im Inneren des Portkörpers, der Portkammer, ist eine durchstechbare Membran angeordnet, welche die beiden Öffnungen trennt. Am Aussenmantel des Portkörpers sind mehrere radiale Rillen, zur seitlichen Verankerung des Ports im subcutanen Hautgewebe angeordnet, wobei sich die äusserste Rille direkt unter der Hautoberfläche befindet. Im Gewebe wird der Portkörper zusätzlich mittels einer Grundplatte verankert.

Ein weiterer zylindrischer, perkutanimplantierbarer Portkörper ist aus der Patentschrift US-A-3 783 868 bekannt. Diese Vorrichtung weist einen zylindrischen Schaft mit einem peripher zur Hautoberfläche abfallenden Verankerungsteller auf. Ein aus der Hautoberfläche ragender Teil ist mit einem Deckel verschließbar und stellt eine Verbindung her zwischen einer nach außen weisenden Öffnung und einer zur Körperinnenseite führenden Katheterleitung.

Die Nachteile des subcutanen Ports bestehen darin, dass man den Katheter weder auswechseln, noch mechanisch reinigen kann, ohne den Port zu explantieren. Ein weiterer Nachteil besteht darin, dass die Haut immer an der gleichen Stelle durchstochen wird. Das ist kurzfristig schmerzhaft und führt längerfristig zur Perforation von Haut und Membran.

Die Nachteile beim erwähnten percutanen Port bestehen darin, dass er sehr schwer und die von aussen sichtbare Fläche sehr gross ist. Zudem ist der aus Metall gefertigte Portkörper auch farblich sehr auffällig. Im montierten Zustand befindet sich ein Luftspalt zwischen der Grundplatte und dem Portkörper, welcher schwierig zu reinigen und zu sterilisieren ist. Hier besteht die Gefahr einer Infektion. Die radialen Rillen sind so angelegt und dimensioniert, dass scharfe Kanten und Ecken entstehen. An diesen Stellen ist ein optimales Einwachsen der Gewebezellen und eine ausreichende Reinigung der Oberfläche nicht möglich. Da keine geometrische Trennlinie zwischen der Hautoberfläche und der obersten Rille vorhanden ist, besteht die Gefahr, dass Körperschweiss oder Schmutz von aussen direkt in die Rillen gelangen. Es kommt im Extremfall zu einer Infektion und der Port muss explantiert werden. Ein weiterer Nachteil besteht darin, dass beim erwähnten Port die Einzelteile aus Vollmaterial in spannender Bearbeitung hergestellt werden müssen. Daher sind die Herstellkosten entsprechend hoch und jegliche Massnahme zur Gewichtsreduktion mit einem Mehraufwand verbunden. Bei den bisherigen Verankerungen besteht weiterhin die Gefahr, dass Teile der Verankerung, durch Einwirkung eines Kippmoments, aus der Haut ragen können.

Hier will die Erfindung Abhilfe verschaffen. Der Erfindung liegt die Aufgabe zugrunde, einen kostengünstigen Port zu entwickeln, dessen körpergerecht geformtes Gehäuse eine zusammenhängende Mantelfläche und fliessende Übergänge aufweist. Der Port soll vorzugsweise im Spritzgussverfahren hergestellt werden und biokompatibel sein. Die Haut muss sich fest an die Portwand klammern können. Die Einwachstiefe der Haut soll möglichst gleichmässig sein und vom Port reguliert werden können. Die Form der Verankerung muss so konzipiert werden, dass im Falle eines Kippmoments keine Kanten oder andere Teile der Verankerung aus dem Körper des Patienten ragen können.

Die Erfindung löst die gestellte Aufgabe mit einem Portkörper, welcher die Merkmale des Anspruchs 1 aufweist.

Die durch die Erfindung erreichten Vorteile sind im wesentlichen darin zu sehen, dass der Portkörper kostengünstiger herzustellen ist, während dem Gebrauch besser gereinigt werden kann, durch die Ausgestaltung der Aussenfläche vom Körper besser gehalten wird und im Falle eines Kippmoments die Haut über die Verankerungsflächen abrollt. Die Wahl des Werkstoffes, die Form und die Oberflächenstruktur des Portkörpers ermöglichen eine längere Implantationszeit.

Ein bevorzugtes Ausführungsbeispiel der Erfindung ist in den Figuren dargestellt.

Es zeigen:
- Fig. 1: einen erfindungsgemässen Portkörper im Querschnitt
- Fig. 2: Einen erfindungsgemässen percutanen Portkörper, im menschlichen oder tierischen Körper angeordnet

Im folgenden bedeuten die Begriffe innen = innerhalb des menschlichen oder tierischen Körpers und aussen = ausserhalb des menschlichen oder tierischen Körpers.

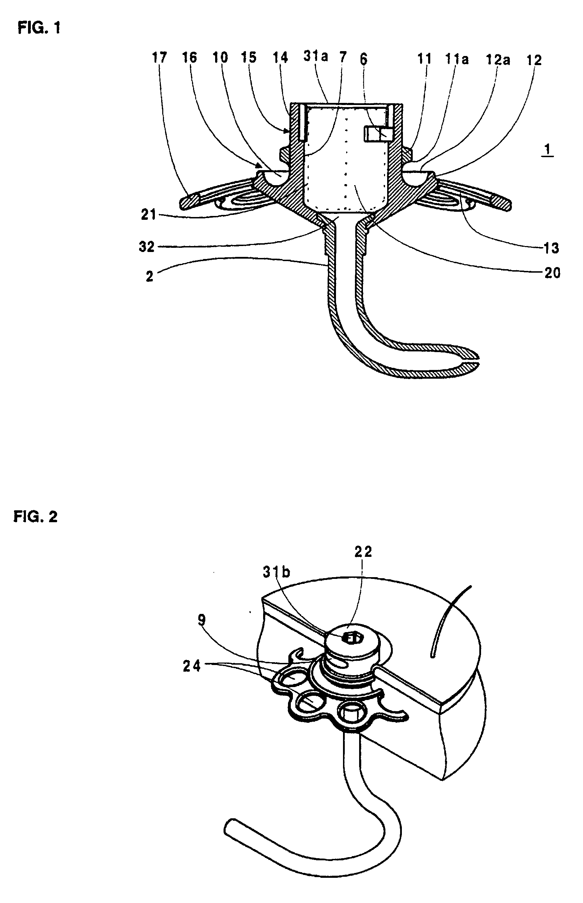

Wie in den Fig. 1 und 2 dargestellt, lässt sich der Portkörper 1 in zwei Hauptelemente einteilen: einen hohlzylindrischen Schaft 14 und einen daran angeordneten radialen Verankerungsteller 13.

Der Portkörper 1 weist zwei gegenüberliegende Öffnungen 31a,32 auf. Die gegen aussen liegende Öffnung 31a entspricht dem Innendurchmesser des zylindrischen Portkörpers 1. Diese Öffnung 31a kann durch einen Deckel 22, in dessen Zentrum sich eine kleinere Öffnung 31b befindet, verkleinert werden. Durch diese kleine, verbleibende Öffnung 31b kann ein Infusionsschlauch ins Innere des Portkörpers geschoben werden. Die zweite, gegen innen liegende Öffnung 32 dient zur Anordnung eines Katheters 2, welcher das zu verabreichende Arzneimittel an den gewünschten Ort im Körperinnern transportiert.

Die Innenwandung 7 des zylindrischen Portkörpers 1 ist im Bereich des Deckels 22 mit Bajonettnocken 6 mit integrierter Verriegelungsnute versehen, so dass der Deckel 22, welcher mit entsprechenden Gegenelementen versehen ist, am Portkörper befestigt werden kann.

Der hohlzylindrische Schaft 14 und der Verankerungsteller 13 werden aus einem einzigen, biologisch verträglichen Kunststoffteil gespritzt. Zwischen den beiden Öffnungen 31b,32 befindet sich eine elastische, selbstschliessende Membran 21, welche die durch den hohlzylindrischen Schaft 14 gebildete Kammer 20 ausfüllt und abdichtet.

Der zylindrische Portkörper 14 wird in zwei Bereiche aufgeteilt, einen gegen aussen liegenden Schaftteil 15 und einen gegen innen liegenden Verankerungsteil 16. Zwischen den beiden Bereichen 15,16 ist eine radial verlaufende, vorstehende Portrippe 11 angeordnet.

Der Schaftteil 15 besteht aus einem inerten Material von glatter Oberflächenstruktur. Er endet gegen aussen mit der gegen aussen liegenden Öffnung 31a und gegen den Verankerungsteil 16 mit der vorstehenden Portrippe 11. In diesem Bereich kann die Haut nicht anwachsen. Von aussen kann man den Schaftteil 15 im implantierten Zustand reinigen bis zur Portrippe 11.

Der Verankerungsteil 16 besteht aus der Portrippe 11, einer vom Verankerungsteil 16 abstehende Verankerungsrippe 12 und dem Verankerungsteller 13. Sowohl die Portrippe 11, wie auch die Verankerungsrippe 12 weisen einen peripheren Rippenrand 11a,12a auf. Die Portrippe 11 und die Verankerungsrippe 12 bilden eine dazwischenliegende kanalförmige, radiale Tasche 10, indem der Abstand zwischen den beiden peripheren Rippenrändern 11a,12a wesentlich enger ist, als der Querschnittdurchmesser der radialen Tasche 10 selbst. Durch den zwischen den beiden Rippenrändern 11a,12a gebildeten Abstand können Gewebezellen in die kanalförmige, radiale Tasche 10 einwachsen.

Die Verankerungsrippe 12 kann Teil des Verankerungstellers 13 sein oder unabhängig von diesem zwischen der Portrippe 11 und dem Verankerungsteller 13 angeordnet werden.

Der Verankerungsteil 16 ist mit einem bioaktiven Material beschichtet und hat eine rauhe Oberflächenstruktur. Dadurch kann sich Gewebe in der Tasche 10 ausbreiten und Gewebezellen können sich an der rauhen Oberfläche festhalten.
Beim Einwachsen von Gewebe in die radiale Tasche 10 verkeilt sich das Gewebe in der radialen Tasche 10 und sorgt für eine formschlüssige Verbindung Gewebe und der Oberfläche der radialen Tasche 10.

Der Verankerungsteller 13 ist radial um den Verankerungsteil 16 des hohlzylindrischen Schafts 14 angeordnet. Der Verankerungsteller 13 weist eine zur Hautoberfläche peripher abfallende Form auf, welche an einen Teller erinnert. Die abfallende Form bewirkt, dass sich die Haut bei Einwirkung eines Kippmoments auf den Portkörper 1 über den Verankerungsteller 13 rollt und nicht von dessen Rand 17 durchbohrt werden kann.

Im Verankerungsteller 13 sind Bohrungen 24 angelegt, welche vom umliegenden Gewebe durchwachsen werden und somit optimalen Halt bieten können.

Damit der Portdeckel 22 im implantierten Zustand geöffnet werden kann, ist am oberen Ende der Aussenfläche des Portschafts 15 eine Montagehilfsaussparung 9 angeordnet. Drei solche Aussparungen 9 auf gleicher Höhe werden von einem Spezialwerkzeug gefasst und der Deckel 22, dessen Öffnung 31b sechskantig ausgeführt ist, wird durch eine Drehbewegung aus der Verbindung mit dem Portkörper 14 gelöst.

Bei einem percutanen Portkörper 1, wird der Deckel 22 vorzugsweise in einer hautähnliche Farbe hergestellt, da diese Fläche gegen aussen sichtbar ist. Selbstverständlich kann auch der ganze Portkörper in einer hautähnlichen Farbe hergestellt werden.

## Patentansprüche

1. Implantierbare zylinderförmige Vorrichtung (1) für die Verbindung eines Schlauches außerhalb des menschlichen oder tierischen Körpers mit einem innerhalb dieses Körpers legbaren Schlauch (2), umfassend einen zylindrischen Schaft (14) und eine um den Schaft (14) angeordnete, radiale und zur Hautoberfläche peripher abfallende Verankerungsplatte (13) **dadurch gekennzeichnet, dass** der zylindrische Schaft (14) wenigstens eine von seinem Außenmantel abstehende, radiale, umlaufende und mit dem Schaft (14) einstückige Rippe (11) aufweist, wobei die wenigstens eine Rippe (11) den Schaft (14) in einen gegen außen liegenden Schaftteil (15) und einen gegen innen liegenden Verankerungsteil (16) aufteilt.

2. Vorrichtung nach Anspruch 1, **dadurch gekennzeichnet, dass** von der Verankerungsplatte (13) wenigstens eine Verankerungsrippe (12) absteht.

3. Vorrichtung nach Anspruch 2, **dadurch gekennzeichnet, dass** die Verankerungsrippe (12) Teil der Verankerungsplatte (13) ist und von dieser absteht.

4. Vorrichtung nach Anspruch 2 oder 3, **dadurch gekennzeichnet, dass** die Verankerungsrippe (12) zwischen der radial abstehenden Rippe (11) und der Verankerungsplatte (13) angeordnet ist.

5. Vorrichtung nach einem der Ansprüche 1 bis 4, **dadurch gekennzeichnet, dass** die radial abstehende Rippe (11) und die Verankerungsrippe (12) jeweils einen peripheren Rippenrand (11a, 12a) aufweisen.

6. Vorrichtung nach Anspruch 5, **dadurch gekennzeichnet, dass** ein Abstand der beiden Rippenränder (11a, 12a) geringer ist als ein Querschnittdurchmesser einer durch die Rippen (11, 12) gebildeten kanalförmigen radialen Tasche (10).

7. Vorrichtung nach einem der Ansprüche 1 bis 6, **dadurch gekennzeichnet, dass** in der Verankerungsplatte (13) Bohrungen (24) angelegt sind.

8. Vorrichtung nach einem der Ansprüche 1 bis 7, **dadurch gekennzeichnet, dass** die Vorrichtung aus einem Kunststoffteil gespritzt werden kann.

9. Vorrichtung nach einem der Ansprüche 1 bis 8, **dadurch gekennzeichnet, dass** die Vorrichtung (1) oder Teile davon eine hautähnliche Farbe haben.

10. Vorrichtung nach einem der Ansprüche 1 bis 9, **dadurch gekennzeichnet, dass** die Vorrichtung (1) vollkommen unter der Haut anordenbar ist.

11. Vorrichtung nach einem der Ansprüche 1 bis 9, **dadurch gekennzeichnet, dass** eine Fläche, welche mit einer Öffnung (31b) versehen ist, außerhalb des Körpers anordenbar ist.

12. Vorrichtung nach einem der Ansprüche 1 bis 11, **dadurch gekennzeichnet, dass** der innerhalb des menschlichen oder tierischen Körpers legbare Schlauch (2) eine Öffnung (32) der Vorrichtung (1) abdichtet und ein Deckel (22), in dessen Zentrum sich eine kleinere Öffnung (31b) befindet, eine zweite Öffnung (31a) der Vorrichtung (1) verkleinert.

13. Vorrichtung nach einem der Ansprüche 1 bis 12, **dadurch gekennzeichnet, dass** im Inneren der Vorrichtung (1) eine durchstossbare, elastische Membran (21) angeordnet ist, welche die zweite Öffnung (31b) abdichtet.

## Claims

1. A cylindrical implant device (1) for connecting a tube outside the human or animal body to a tube (2) which may be placed inside said body, comprising a cylindrical shaft (14) and a radial anchoring plate (13) arranged around said shaft (14) and tapering off peripherally to the surface of the skin, wherein the cylindrical shaft (14) comprises at least one radial, circumferential rib (11) projecting from its outer surface and formed integrally with the shaft (14), and said at least one rib (11) divides the shaft (14) into a shaft portion (15) lying outwards and an anchoring portion (16) lying inwards.

2. The device as set forth in claim 1, wherein at least one anchoring rib (12) projects from said anchoring plate (13).

3. The device as set forth in claim 2, wherein said anchoring rib (12) is a part of the anchoring plate (13) and projects from it.

4. The device as set forth in claim 2 or 3, wherein the anchoring rib (12) is arranged between the radially projecting rib (11) and the anchoring plate (13).

5. The device as set forth in any one of claims 1 to 4, wherein the radially projecting rib (11) and the anchoring rib (12) each comprise a peripheral rib edge (11a, 12a).

6. The device as set forth in claim 5, wherein a distance between the two rib edges (11a, 12a) is smaller than a cross-sectional diameter of a channel-shaped, radial pocket (10) formed by the ribs (11, 12).

7. The device as set forth in any one of claims 1 to 6, wherein bores (24) are disposed in the anchoring plate (13).

8. The device as set forth in any one of claims 1 to 7, wherein said device can be injected from a plastic part.

9. The device as set forth in any one of claims 1 to 8, wherein the device (1) or parts of it have a skin-like colour.

10. The device as set forth in any one of claims 1 to 9, wherein the device (1) may be arranged completely under the skin.

11. The device as set forth in any one of claims 1 to 9, wherein a surface provided with a port (31b) may be arranged outside the body.

12. The device as set forth in any one of claims 1 to 11, wherein the tube (2) which may be placed inside the human or animal body seals off a port (32) of the device (1) and reduces a second port (31a) of the device (1), at the centre of which a smaller port (31b) is situated.

13. The device as set forth in any one of claims 1 to 12, wherein in the interior of the device (1), a penetrable, elastic membrane (21) is arranged which seals off the second port (31a).

## Revendications

1. Dispositif cylindrique implantable (1), destiné au raccordement d'un flexible à l'extérieur du corps humain ou animal, comportant un flexible (2) pouvant être implanté à l'intérieur dudit corps, comprenant un fût cylindrique (14) et un disque radial d'ancrage (13) qui est disposé autour dudit fût (14) et décline périphériquement vers la surface de la peau, **caractérisé par le fait que** le fût cylindrique (14) possède au moins une nervure radiale périphérique (11) qui forme un seul tenant avec ledit fût (14), au-delà de l'enveloppe extérieure duquel elle fait saillie, ladite nervure (11), prévue au minimum, scindant ledit fût (14) en une partie longiforme (15) située vers l'extérieur et en une partie d'ancrage (16) située vers l'intérieur.

2. Dispositif selon la revendication 1, **caractérisé par le fait qu'**au moins une nervure d'ancrage (12) fait saillie au-delà du disque d'ancrage (13).

3. Dispositif selon la revendication 2, **caractérisé par le fait que** la nervure d'ancrage (12) fait partie intégrante du disque d'ancrage (13), ou fait saillie au-delà de ce dernier.

4. Dispositif selon la revendication 2 ou 3, **caractérisé par le fait que** la nervure d'ancrage (12) est interposée entre le disque d'ancrage (13) et la nervure (11) saillant radialement.

5. Dispositif selon l'une des revendications 1 à 4, **caractérisé par le fait que** la nervure (11) saillant radialement, et la nervure d'ancrage (12), sont respectivement pourvues d'un bord périphérique (11a, 12a).

6. Dispositif selon la revendication 5, **caractérisé par le fait qu'**une distance, séparant les deux bords (11a, 12a) des nervures, est plus petite qu'un diamètre de section transversale d'une poche radiale (10) en forme de canal, matérialisée par lesdites nervures (11, 12).

7. Dispositif selon l'une des revendications 1 à 6, **caractérisé par le fait que** des perçages (24) sont pratiqués dans le disque d'ancrage (13).

8. Dispositif selon l'une des revendications 1 à 7, **caractérisé par le fait que** ledit dispositif peut être moulé par injection, sous la forme d'une pièce en matière plastique.

9. Dispositif selon l'une des revendications 1 à 8, **caractérisé par le fait que** ledit dispositif (1), ou des parties de celui-ci, présente(nt) une couleur analogue à celle de la peau.

10. Dispositif selon l'une des revendications 1 à 9, **caractérisé par le fait que** ledit dispositif (1) peut être intégralement placé au-dessous de la peau.

11. Dispositif selon l'une des revendications 1 à 9, **caractérisé par le fait qu'**une surface, pourvue d'un orifice (31b), peut être disposée à l'extérieur du corps.

12. Dispositif selon l'une des revendications 1 à 11, **caractérisé par le fait que** le flexible (2), pouvant être implanté à l'intérieur du corps humain ou animal, assure l'étanchéité d'un orifice (32) dudit dispositif (1) ; et un couvercle (22), au centre duquel se trouve un orifice (31b) plus petit, provoque la réduction d'un second orifice (31a) du dispositif (1).

13. Dispositif selon l'une des revendications 1 à 12, **caractérisé par le fait qu'**une membrane élastique perforable (21), logée à l'intérieur dudit dispositif (1), assure l'étanchéité du second orifice (31b)
